# EUROPEAN PATENT APPLICATION

(11) **EP 1 764 119 A1**
(43) Date of publication of application: **21.03.2007**
(21) Application number: 05019717.7
(22) Date of filing: 09.09.2005
(51) Int. Cl.: A61L 31/10, A61L 31/16, A61L 27/54, A61K 33/00

(54) **Implants with improved osteointegration**

(71) Applicant: NOLabs AB, 252 21 Helsingborg (SE)
(72) Inventor: Peters, Tor, 8703 Erlenbach (CH)
(74) Representative: Asketorp, Göran

(57) **Abstract**

A coating on an implant, said implant being intended for implantation in/on an implantation area, is provided. The coating comprises nitric oxide (NO) for obtaining an anti-viral, anti-fungal, and anti-bacterial effect, and for promotion of osteo-integration of the implant, bone healing, bone growth, and wound healing at said implantation area. An implant and a kit of implants, comprising said coating are also provided.

## Description

### Field of the Invention

This invention pertains in general to the field of a coating of an implant, said implant being configured for surgical treatment of fractures, deformities, tumour diseases, replacement of tissue, such as bone, and promotion of osteo-integration and wound-healing of the implant, said coating involving the use of nitric oxide (NO). More particularly the present invention pertains to a kit of such coated implants.

### Background of the Invention

In the field of implant surgery, surgeons implant a wide variety of metallic, ceramic, and polymeric materials into patients, such as humans or animals. Surgeons use these kind of implants for orthopaedic purposes, such as treatment of fractures, treatment of deformities, tumour diseases, and replacement of tissue, such as bone, but also in other fields of implantation, such as cosmetic surgery, reconstructive surgery, wire leads, heart surgery, such as heart valve surgery, aneurysm clips, and dental surgery.

A problem associated with insertion of implants is viral and bacteriological infection, caused by virus, fungi, and/or bacteria that get access to the tissue in the vicinity of the inserted implant, when the body of the patient is opened, or when a wound is inflicted during trauma. It is also possible that the implant in itself carries virus, fungi, or bacteria.

Also, the body of the patient, in which the implant has been inserted, recognises implants as foreign objects, possibly leading to local and systemic reactions. Thus, a problem in prior art is osteo-integration of the implants.

Even if bone is hard and strong enough to support the weight of our bodies, it is by no means an unchangeable tissue. Living cells account for about 15% of the weight of compact bone, and these cells are engaged in an unceasing process of remodelling. One class of cells (osteoclasts) destroys old bone matrix while another (osteoblasts) deposits new bone matrix. This mechanism provides for continuous turnover and replacement of the bone matrix in the interior of the bone through which it can adapt to the load it bears. This is also a prerequisite to successful osteo-integration of implants.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic in high concentrations and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthylenelmine) and B-PEI (Branched PolyEthyleneImine), which polymers have the advantage of being biocompatible with natural products, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of a coating of an NO eluting polymer on implants to provide an anti-bacterial, anti-fungi, and anti-viral effect, by elution of nitric oxide NO.

Thus, it would be appreciated to provide a way of obtaining an anti-viral, anti-fungal, and anti-bacterial effect, while simultaneously obtaining promotion of osteo-integration of the implant, bone healing, bone growth, and wound healing.

### Summary of the Invention

Accordingly, the present invention preferably seeks to mitigate, alleviate or eliminate one or more of the above-identified deficiencies in the art and disadvantages singly or in any combination and solves at least the problems mentioned above, at least partly by providing a coating, an implant, and a kit of implants, according to the appended patent claims.

According to one aspect of the invention, a coating is provided, which coating allows for anti-viral, anti-fungal, and anti-bacterial effect, and promotion of osteo-integration of the implant, bone healing, bone growth, and wound healing, on an implant. Said coating comprises a nitric oxide (NO) eluting polymer, such that a therapeutic dose of nitric oxide is eluted from said nitric oxide eluting polymer, allowing for anti-viral, anti-fungal, and anti-bacterial effect, and promotion of osteo-integration of the implant, bone healing, bone growth, and wound healing.

According to another aspect of the invention, an implant is provided, which implant has at least partly said coating.

According to still another aspect of the invention a kit of said implants is provided.

The present invention has at least the advantage over the prior art that it provides target exposure of a tissue or organ in the vicinity of an implant to NO, whereby an increased circulation in the tissue or organ area, anti-viral, anti-fungal, and anti-bacterial effect, and promotion of osteo-integration of the implant, bone healing, bone growth, and wound healing, while not developing resistance against the active pharmaceutical substance, pain etc, simultaneously are obtained.

### Brief Description of the Drawing

These and other aspects, features and advantages of which the invention is capable of will be apparent and elucidated from the following description of embodiments of the present invention, reference being made to the accompanying drawing, in which

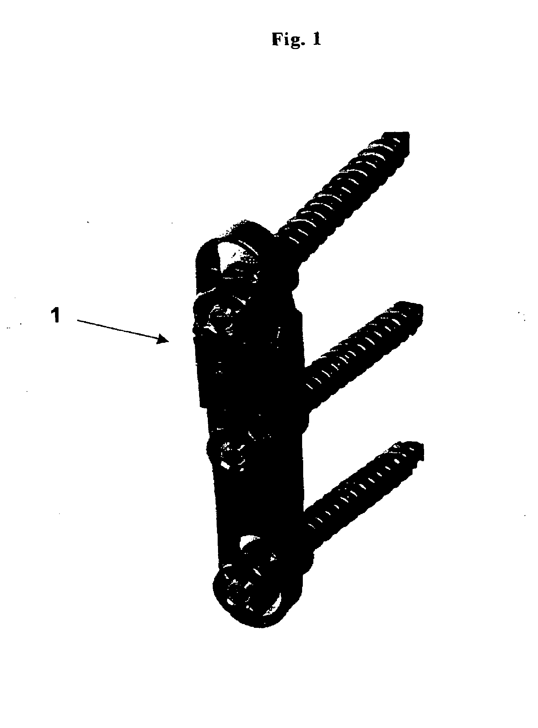

Fig. 1 is an illustration of one example of an implant according to an embodiment of the present invention.

### Description of Embodiments

The following description focuses on embodiments of the present invention applicable to a coating on implants, which coating allows for anti-viral, anti-fungal, and anti-bacterial effect, and promotion of osteo-integration of the implant, bone healing, bone growth, and wound healing.

The patient according to the embodiments may be a human or animal, such as mammals selected from the group consisting of cat, dog, horse, cattle etc.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, i.e. 100 to 1000 folded higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

It has now been shown that NO is an important local mediator of bone cell activity. Changes in the mechanical forces acting on bone lead to adaptive remodelling of the bone. NO is an important signalling molecule on mature bone tissue, triggering the adaptive response.

Osteoblasts and osteclasts both produce and respond to NO; low doses of NO support and higher doses inhibit osteoclast and osteoblast function.

All three types of NOS are involved in the development and homeostasis of bone tissue. Basal low-level NO synthesis by eNOS and nNOS stimulates osteoblasts and osteoclasts, respectively, and is essential for their function. Lack of eNOS results in reduced bone formation and bone volume. ENOS-deficient osteoblasts also show weaker response to the growth factor TGF-beta that is necessary for the requirement of osteoblasts to remodelling sites. nNOS-deficiency, on the other hand, show defective bone turn-over.

Exogenous NO in still higher concentrations inhibits bone resorption by suppressing the formation and activity of osteoclasts.

The present invention takes advantage of these facts and therefore presents an unexpected effect in respect of osteo-integration of implants by using the NO eluting coating according to the present invention on implants.

In recent years research has been directed to polymers with the capability of releasing nitrogen oxide when getting in contact with water. Such polymers are for example polyalkyleneimines, such as L-PEI (Linear PolyEthylenelmine) and B-PEI (Branched PolyEthyleneImine), which polymers have the advantage of being biocompatible, after the release of nitrogen oxide.

The polymers employed in embodiments of the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6, 520, 425, and US 6,695,992 disclose processes and apparatuses for the production of such polymeric fibres. These techniques are generally based on gas stream spinning, also known within the fiber forming industry as air spinning, of liquids and/or solutions capable of forming fibers. Gas stream spinning is suited for producing devices according to certain embodiments of the invention.

In an embodiment of the invention an NO eluting polymer is electro spun onto an implant. The implant may, according to different embodiments, for example be a temporary, a permanent, or biodegradable implant. Temporary implants are implants that are removed after a certain time period of implantation. For instance a per se known device 1 comprising screws and/or plates, as shown in Fig. 1, is fixed to a fractured bone across the fracture site thereof. The device is however provided with a coating eluting NO during a certain time after implantation of the device 1. Thus for instance osteo-integration is promoted and the fracture bone heals faster than in the case where device 1 does not have such an advantageous coating. After healing is at least partly achieved, e.g. when the bone fracture has healed to sufficient stability, the temporary device 1 is removed by surgery. Alternative embodiments of biodegradable implants have the ability to break down, safely and relatively quickly, by biological means, into the raw materials of nature and disappear from the body where they were implanted in. In the latter case, the coating eluting NO during a certain time after implantation is also biodegradable or at least biocompatible.

The implant according to an embodiment of the invention is an orthopaedic implant, such as (i) a hip joint, (ii) screws, cannulated screws, nails, intramedullary nails, and plates intended to join or attach bone fragments, pieces, or parts with each other, (iii) external fixators, (iv) implants intended for treatment of degenerative instabilities, fractures, tumours, and deformities in respect of the spine, (v) cranio-maxillofacial implants intended for treatment of fractures, reconstruction, and correction of deformities, of mandible, mid-face, or skull.

In other embodiments the implant may be chosen from the group : 1) dental implants and sealing caps, that are temporarily put over e.g. a titanium screw before an artificial tooth is mounted on the titanium screw, 2) internal and external wound closure, 3) cosmetic surgery, 4) reconstructive surgery, 5) wire leads, 6) heart surgery, such as heart valve surgery, 7) aneurysm clips, 8) ear implants, such as drainage tubes through the eardrum during infection, 9) infusion systems, such as cytostatic infusion systems, 10) stomia systems, such as colostomy, tracheotomy tubes and systems, and 11) tear channel implants.

After the implant, according to above, has been coated with an NO eluting polymer the implant may be mounted, placed, or applied on the area in need of implantation. When the coated implant is in place and gets in contact with the inevitable moisture or water in the body, in which the implant has been implanted, the NO eluting polymer in the coating of the implant starts to elute NO.

In another embodiment of the present invention the implant is partially covered with NO eluting polymer. This embodiment may for example be used when only a part of the implant that is inside the subject body, such as in respect of fixation means for holding a head, vertebra, or knee in a position, which position, for some reason, needs regulation during the healing process. It is of course also within the scope of the present invention to cover the entire implant in these cases with the NO eluting coating, but it would be more economically to only cover the part in contact with the subject body, i.e. a target area.

The elution of NO then brings about an anti-viral, anti-fungal, and anti-bacterial effect, and promotion of osteo-integration of the implant, bone healing, bone growth, and wound healing on the target area.

In another embodiment the NO eluting polymer is co-spun together with another polymer, or other polymers, onto the implant. "Co-spun" in the present context is intended to be interpreted as spun, as a polymer mixture, together with the NO eluting polymer, either by air-spinning or electro spinning. This/these other polymer/polymers may for example be chosen from the group: polyurethane, polyvinylacetates, polylacticacids, starch, cellulose, polyhydroxyalkanoates, polyesters, polycaprolactone, polyvinylalcohol, protein based plastics, gelatine, biogradable polymers, and latex.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of permanently implanted medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

In still another embodiment the NO eluting polymer, according to above, is ground or milled into nano-particles or micro-spheres. These nano-particles or micro-spheres are then applied on the implant by any convenient method, which method is known by the skilled artisan, such as gluing with a glue that not is dissolvable in the body environment of the implant. It is also possible to mix or encapsulate fibres, nano-particles, or micro-spheres of NO eluting polymer with other polymers, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics. When the fibres, nano-particles, or micro-spheres of NO eluting polymer, according to this embodiment, gets in contact with the moisture or water in the implantation area, elution of NO starts and an anti-viral, anti-fungal, and anti-bacterial effect is obtained. This embodiment presents the advantage of controling or regulating the time span of NO release from the implant, by the mixing of other polymers that do not elute NO.

In still another embodiment the NO eluting polymer is integrated in a film of another suitable polymer, polyurethane, or polyethylene, film, which film then is glued on the implant under the restrictions mentioned above. When these film, including NO eluting polymer, gets in contact with the moisture or water in the implantation area, elution of NO starts and an anti-viral, anti-fungal, and anti-bacterial effect, and promotion of osteo-integration of the implant, bone healing, bone growth, and wound healing is obtained.

In another embodiment the nano-particles, or micro-spheres according to above, may be integrated in a soluble film that disintegrates on the implantation area, in order to elute NO at the area of interest when the soluble film gets in contact with the moisture or water in the implantation area.

The coating according to the present invention elutes nitric oxide (NO) from said eluting polymer in a non toxic dose, such as between 0.001 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

The NO-eluting polymers in the coating according to the present invention may be combined with silver, such as hydroactivated silver. The integration of silver in the devices according to the present invention gives the antimicrobial and anti-viral effect an extra boost. Preferably the silver is releasable from the devices in the form of silver ions.

In yet another embodiment of the present invention the NO-eluting coating is acting as a booster for drug eluting patches, e.g. pharmaceuticals, vitamins, nicotin, nitroglycerin, etambutol, Non-Steroidal Anti-Inflammatory Drugs (NSAID), such as diclofenac, ibuprofen, aspirin, naproxen, COX-2 inhibitors, choline magnesium trisalicylate, diflunisal, salsalate, fenoprofen, flurbiprofen, ketoprofen, oxaprozin, indomethacin, sulindac, tolmetin, meloxicam, piroxicam, meclofenamate, mefenamic acid, nabumetone, etodalac, ketorolac, celecoxib, valdecoxib, and rofecoxib; steroids, such as cortisone, prednisone, methylprednisolone, prednisolone, vitamin D, estrogen, cholestrol, beclomethasone, flunisolide, fluticasone, triamcinolone, desonide, clobetasol, alclometasole, desoximetasone, betamethasone, halcinonide and dexamethasone; pain reliefs, such as motrin, feldene, naprosyn, lidocaine, and prilocaine; and other substances, such as indinavirsulfate, finasteride, aprepitant, montelukast sodium, alendronate sodium, rofecoxib, rizatriptan benzoate, simvastatin, finasteride, ezetimibe, caspofungin acetate, ertapenem sodium, dorzolamide hydrochloride, timolol maleate, losartan potassium, and hydrochlorotiazide; etc. This embodiment presents a coating with the advantage of combining two treatments, of significant value, in one treatment.

The device according to the present invention may be manufactured by, for example electro spinning of for example L-PEI. L-PEI is then charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

It is of course possible to electro spin the other NO-eluting polymers, according to above, on the implant while still being inside the scope of the present invention as defined by the appended claims.

In one embodiment the NO-eluting polymers employed in the coating according to the present invention are electro spun in such way that pure NO-eluting polymer fibres may be obtained.

Gas stream spinning, or air-spinning, of said NO-eluting polymers onto the implant is also within the scope of the present invention.

The manufacturing process according to the present invention presents the advantages of large contact surface of the NO-eluting polymer fibres or micro particles with the area to be covered with the coating, effective use of NO-eluting polymer, and a cost effective way of coating the implant.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A coating of an implant, said implant being intended for implantation in/on an implantation area,
wherein
said coating is configured to elute Nitric Oxide (NO) for obtaining an anti-viral, anti-fungal, and anti-bacterial effect, and for promotion of osteo-integration of the implant, bone healing, bone growth, and wound healing at said implantation area, wherein
said coating covers said implant at least partly.

2. The coating according to claim 1, wherein said coating comprises a polymer configured to elute non-toxic dosage of said Nitric Oxide (NO) for obtaining said effects and said promotion when used at said implantation area,
wherein the non-toxic dosage of NO is chosen to support osteoclast and osteoblast function.

3. The coating according to claim 1, wherein said polymer is selected from the group consisting of branched and linear polyalkyleneimines, polyalkyleneimine celluloses, S-nitrosylated polymer, and poly(alkylenimine)diazeniumdiolates, or any combination of these.

4. The coating according to claim 1, wherein said polymer is L-PEI (linear polyethyleneimine), loaded with nitric oxide(NO), arranged for release of the nitric oxide (NO) at said implantation area in, or on, a body of a human or animal.

5. The coating according to claim 1, wherein said coating is at least partly disintegrable when subjected to moisture or water.

6. The coating according to claim 2, wherein said polymer is in form of nano-particles or micro-spheres.

7. Coating according to claim 6, wherein said nano-particles, or micro-spheres, are encapsulated in suitable material, such as polyvinylacetates, polylacticacids, starch, cellulose, polyhydroxyalkanoates, polyesters, polycaprolactone, polyvinylalcohol, protein based plastics, and/or gelatine.

8. Coating according to claim 2, wherein said NO-eluting polymer is applied on, or integrated with, a material selected from the group consisting of polyesters, polyamides, polyethers, polyurethanes, polycarbonates, polyvinylacetates, polylacticacids, starch, cellulose, polyhydroxyalkanoates, polyesters, polycaprolactone, polyvinylalcohol, cotton, polypropylene, polyacrylonitrile, polystyrene, poly(acrylic acid), protein based plastics, and gelatine, or combinations thereof.

9. Coating according to claim 1, wherein said coating comprises silver, configured for exposure of said area.

10. Coating according to claim 1, wherein said coating is configured to act as a booster for other active ingredients chosen from the group consisting of pharmaceuticals, vitamins, nicotin, nitroglycerin, Non-Steroidal Anti-Inflammatory Drugs, steroids, and/or pain reliefs.

11. An implant, comprising the coating according to claim 1.

12. The implant according to claim 11, wherein said implant is an orthopaedic implant, such as (i) a hip joint, (ii) screws, cannulated screws, nails, intramedullary nails, and plates intended to join or attach bone fragments, pieces, or parts with each other, (iii) external fixators, (iv) implants intended for treatment of degenerative instabilities, fractures, tumours, and deformities in respect of the spine, or (v) cranio-maxillofacial implants intended for treatment of fractures, reconstruction, and correction of deformities, of mandible, mid-face, or skull.

13. The implant according to claim 11, wherein said implant is selected from the group: **1**) dental implants and sealing caps, that are temporarily put over the titanium screw before an artificial tooth is mounted on the titanium screw, **2**) internal and external wound closure, **3**) cosmetic surgery, **4**) reconstructive surgery, **5**) wire leads, **6**) heart surgery, such as heart valve surgery, **7**) aneurysm clips, **8**) ear implants, such as drainage tubes through the eardrum during infection, **9**) infusion systems, such as cytostatic infusion systems, **10**) stomia systems, such as colostomy, tracheotomy tubes and systems, and **11**) tear channel implants.

14. A kit of implants according to claim 11, wherein said implants comprise the coating according to claim 1.

15. The kit according to claim 14, wherein said kit comprises screws and plates intended to join or attach bone fragments, pieces, or parts with each other.

16. The kit according to claim 14, wherein said kit comprises temporary implants, bio-degradable and/or non-bio-degradable implants.

17. A process for applying a coating according to claim 1 on an implant according to claim 11, comprising:
selecting a plurality of nitric oxide eluting polymeric particles, preferably nano fibres, nano particles or micro spheres, and
deploying said nitric oxide eluting particles as a coating on said implant,
wherein said deploying comprises electro, air, or gas stream spinning of said particles.

18. Use of a nitric oxide (NO) eluting polymer for the manufacture of a coating on an implant, said implant being intended for implantation in/on an implantation area, wherein
nitric oxide is loaded to said coating, which coating elutes nitric oxide (NO) from said eluting polymer in a non-toxic dose when used in/on said implantation area for obtaining an anti-viral, anti-fungal, and anti-bacterial effect, and for promotion of osteo-integration of the implant, bone healing, bone growth, and wound healing at said implantation area.

19. Use according to claim 18, wherein said non-toxic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
